# EUROPEAN PATENT APPLICATION

(11) **EP 3 632 293 A2**
(43) Date of publication of application: **08.04.2020**
(21) Application number: 19200767.2
(22) Date of filing: 01.10.2019
(51) Int. Cl.: A61B 1/00, A61B 1/12, A61B 1/313

(54) **MULTI LUMEN ACCESS DEVICE**

(30) Priority: 02.10.2018 US 201816149479
(71) Applicant: Covidien LP, Mansfield, MA 02048 (US)
(72) Inventor: HOLSTEN, Henry E., Hamden, CT 06518 (US)
(74) Representative: Maschio, Antonio

(57) **Abstract**

A surgical access device includes a housing, a tubular member extending from the housing, a valve disposed on the housing, and a tip member at a distal end of the tubular member. The housing includes a seal and the tubular member includes a plurality of lumens extending therethrough. The valve is fluidly coupled with a first lumen of the plurality of lumens and the tip member includes a first port that is aligned and fluidly coupled with the first lumen of the plurality of lumens. The first port is configured to direct a fluid towards a predetermined location.

## Description

### TECHNICAL FIELD

The present disclosure relates to a surgical access device. More particularly, the present disclosure relates to a surgical access device having multiple lumens.

### BACKGROUND OF RELATED ART

Minimally invasive surgery has become increasingly popular in recent years. Minimally invasive surgery eliminates the need to cut a large incision in a patient, thereby reducing discomfort, recovery time, and many of the deleterious side effects associated with traditional open surgery. Minimally invasive viewing instruments (e.g., laparoscopes and endoscopes) are optical instruments to facilitate the viewing of internal tissues and/or organs.

Laparoscopic surgery involves the placement of a laparoscope in a small incision in the abdominal wall of a patient to view the surgical site. Endoscopic surgery involves the placement of an endoscope in a naturally occurring orifice (e.g., mouth, nose, anus, urethra, or vagina) to view the surgical site. Other minimally invasive surgical procedures include video assisted thoracic surgery and cardiovascular surgery conducted through small incisions between the ribs. These procedures also utilize scopes to view the surgical site.

A typical minimally invasive viewing instrument (e.g., a laparoscope or an endoscope) includes a housing, an elongated shaft extending from one end of the housing, and a lens that is provided in the distal end of the shaft. A camera viewfinder extends from the other end of the housing. A camera is connected to the housing and transmits images of the surgical field viewed through the lens to a monitor on which the images are displayed. During a surgical procedure, the distal end portion of the shaft is extended into the patient, while the proximal end portion of the shaft, the housing, and the camera viewfinder remain outside the patient. In this manner, the laparoscope/endoscope is positioned and adjusted to view particular anatomical structures in the surgical field on the monitor.

During insertion of an endoscope or a laparoscope into the body and during the surgical procedure, debris (e.g., organic matter and moisture) may be deposited on the lens of the endoscope. The buildup of debris and condensation on the lens impairs visualization of the surgical site, and often necessitates cleaning of the lens. This may require the surgeon to remove, clean, and re-insert the endoscope one or more times during a surgical procedure to maintain a clear image of the surgical site. Cleaning of the instruments often necessitates removal of the instruments from the surgical site, thereby increasing the time required to perform the surgical procedure.

Systems for cleaning viewing devices such as endoscopes and laparoscopes are known in the art. Examples of known systems and techniques are described in U.S. Patent Application Publication No. 2009/0234193 to Weisenburgh, II et al., U.S. Patent No. 8,047,215 to Sasaki, and U.S. Patent No. 8,888,689 to Poll et al.

### SUMMARY

According to one embodiment of the present disclosure, a surgical access device includes a housing including a seal, a tubular member extending from the housing, the tubular member including a plurality of lumens extending therethrough, a valve disposed on the housing and fluidly coupled with a first lumen of the plurality of lumens, and a tip member disposed at a distal end of the tubular member, the tip member including a first port that is aligned and fluidly coupled with the first lumen of the plurality of lumens, the first port configured to direct a fluid towards a predetermined location.

The surgical access device may include the tubular member with an inner tube and an outer tube defining an annular chamber therebetween. The annular chamber may be fluidly coupled to the valve and the first lumen of the plurality of lumens is disposed within the annular chamber.

The surgical access device may include the annular chamber having the second lumen of the plurality of lumens extending therethrough. The second lumen of the plurality of lumens may be fluidly coupled to a second port located in the tip member. The second port may be configured to direct a fluid towards the predetermined location.

The surgical access device of may include the inner tubular member defining a third lumen of the plurality of lumens extending therethrough.

The surgical access device may include the first and second lumens of the plurality of lumens being radially spaced apart.

The surgical access device may include the predetermined location lying along a central longitudinal axis of the tubular member.

The surgical access device may include the valve fluidly coupling a source of fluid to the first and second lumens of the plurality of lumens.

The surgical access device may include the first port being offset from the second port by 180°.

The surgical access device may include each of the first and second ports having a spray pattern that covers 180° of the predetermined location.

The surgical access device may include the first port and the second port being radially offset in a range between about 60° and about 120°.

The surgical access device may include the channel being configured to receive a viewing instrument therethrough.

The surgical access device may be insertable through an opening in tissue.

According to an embodiment of the present disclosure, a method for cleaning a viewing instrument includes moving a lens of a viewing instrument towards a target area defined in a channel of a tubular member, the tubular member including an inner tube disposed in an outer tube defining an annular chamber therebetween, and dispensing a cleaning fluid from a first port towards the target area, the first port located on a tip member, the tip member located at a distal end of the tubular member, the first port fluidly coupled to a first lumen of a plurality of lumens that is disposed in the annular chamber, the first lumen of the plurality of lumens fluidly coupled to a valve for controlling flow of the cleaning fluid.

The method may include dispensing the cleaning fluid from a second port towards the target area. The second port may be located on the tip member and fluidly coupled to a second lumen of the plurality of lumens that is disposed in the annular chamber. The second lumen of the plurality of lumens may be fluidly coupled to the valve for controlling flow of the cleaning fluid.

The method may include moving the optical portion into a third lumen of the plurality of lumens defined by the inner tube.

The method may further include positioning the tubular member through tissue of a patient. The tubular member may extend from a housing with a seal member.

The method may further include repositioning the lens of the viewing instrument along a longitudinal axis of the tubular member such that the lens moves into and out of the predetermined region.

The method may further include viewing an image on a monitor coupled to the viewing instrument during repositioning of the lens.

### BRIEF DESCRIPTION OF THE DRAWINGS

Various embodiments of the present disclosure are illustrated herein with reference to the accompanying drawings, wherein:
FIG. 1 is a perspective view of a surgical access device coupled to a source of fluid according to an embodiment of the present disclosure;
FIG. 2 is an exploded, perspective view of the surgical access device of FIG. 1 with parts separated;
FIG. 3 is a perspective view of a tubular member of the surgical access device of FIG. 1 shown in phantom;
FIG. 4 is an enlarged view of the indicated area of detail of FIG. 2;
FIG. 5 is an enlarged view of the indicated area of detail of FIG. 2;
FIG. 6 is a perspective view of a distal tip of the surgical access device of FIG. 1;
FIG. 7 is a side cross-sectional view of the surgical access device of FIG. 1 taken along section line 7-7 in FIG. 1;
FIG. 8 is an enlarged view of the indicated area of detail of FIG. 7;
FIG. 9 is an enlarged view of the indicated area of detail of FIG. 7;
FIG. 10 is an end, cross-sectional view of the surgical access device of FIG. 7 taken along section line 10-10 in FIG. 7;
FIG. 11 is a cross-sectional view of the surgical access device of FIG. 7 taken along section line 11-11 in FIG. 7;
FIG. 12 is an end cross-sectional view of the distal tip of the surgical access device of FIG. 11 taken along section line 12-12 in FIG. 11; and
FIG. 13 is a perspective view of an endoscope.

### DETAILED DESCRIPTION

Embodiments of the presently disclosed surgical access device are described in detail with reference to the drawings, wherein like reference numerals designate corresponding elements in each of the several views. As used herein, the term "distal" refers to that portion of the instrument, or component thereof which is farther from the user while the term "proximal" refers to that portion of the instrument or component thereof which is closer to the user.

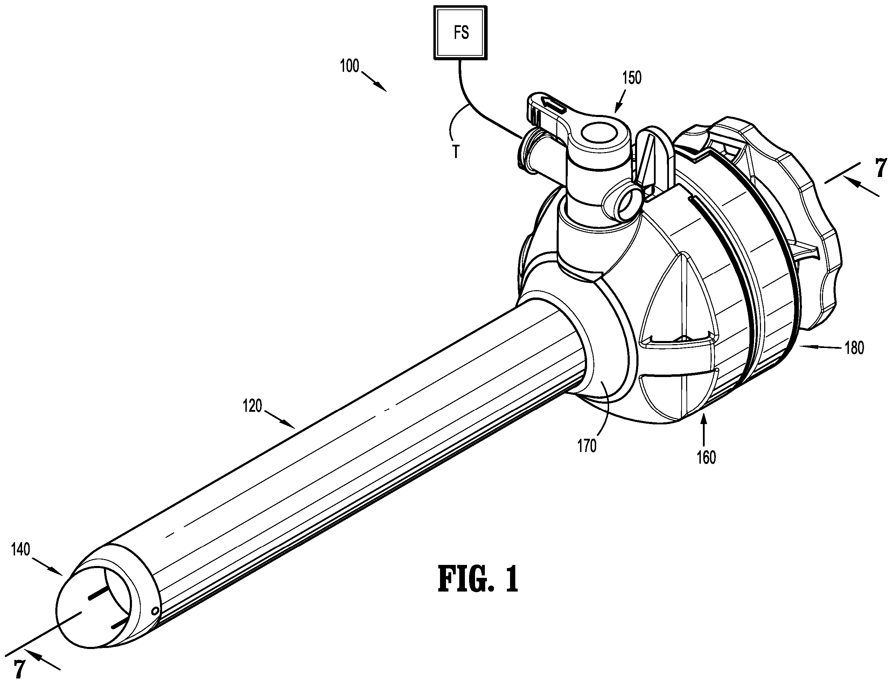

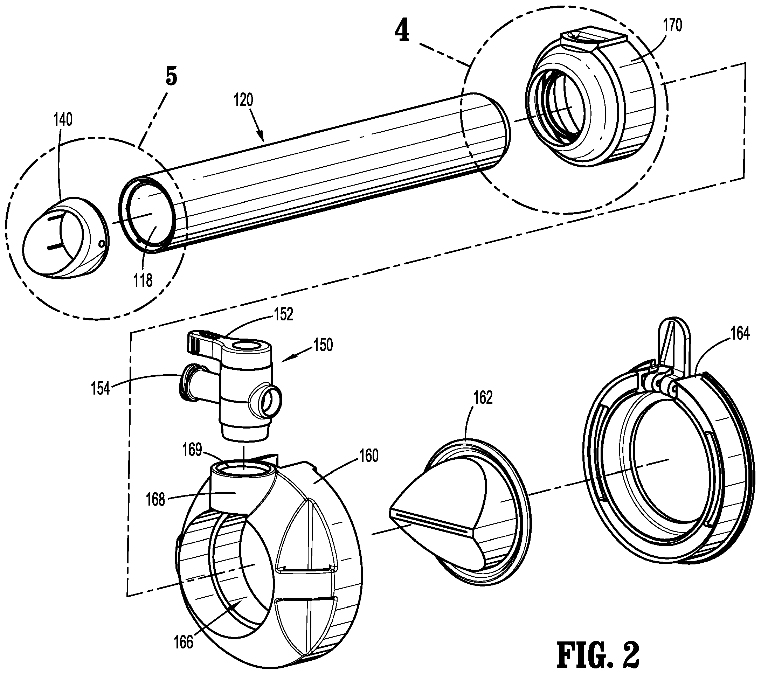

Various embodiments of a surgical access device are described herein. With initial reference to FIGS. 1 and 2, a surgical access device 100 is illustrated. The components of the surgical access device 100 may be formed from suitable biocompatible materials such as medical grade metals (e.g., stainless steel), polymeric materials (e.g., polycarbonate), or combinations thereof. The surgical access device 100 includes a housing 160. A collar 170 is insertable through the housing 160 and a tubular member 120 extends from a distal end of the collar 170. A tip member 140 is located at a distal end of the tubular member 120. A seal assembly 180 is releasably coupled to a proximal end of the housing 160. An example of a suitable seal assembly usable with the presently disclosed surgical access device 100 is described in U.S. Patent No. 10,022,149, issued on July 17, 2018, the entire contents of which are hereby incorporated by reference. It is contemplated that the tubular member 120 may include a plurality of spaced annular ribs along a portion of a length of the tubular member to improve retention of the surgical access device 100 in an opening through body tissue. An example of a cannula with annular ribs is disclosed in U.S. Patent No. 8,740,925, issued on June 3, 2014, the entire contents of which are hereby incorporated by reference. Additionally, the surgical access device 100 may include a fixation device such as a balloon, an umbrella, a foam collar, etc. An example of a surgical access device with a foam collar and an anchoring balloon is disclosed in U.S. Patent No. 7,963,975, issued on June 21, 2011, the entire contents of which are hereby incorporated by reference. The surgical access device 100 may include a combination of ribs, balloons, foam collars, or other known structures for securing an access device in body tissue. Access devices with other fixation features are disclosed in U.S. application no. 62/631,540, U.S. application no. 16/043,279, U.S. application no. 62/653,859, and U.S. application no. 62/568,497, the entire disclosures of which are hereby incorporated by reference herein.

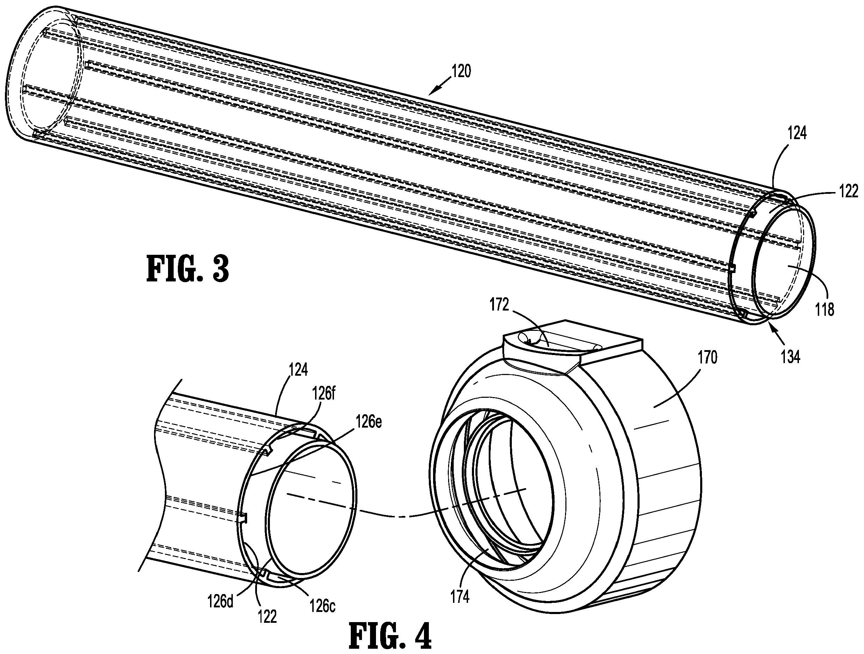

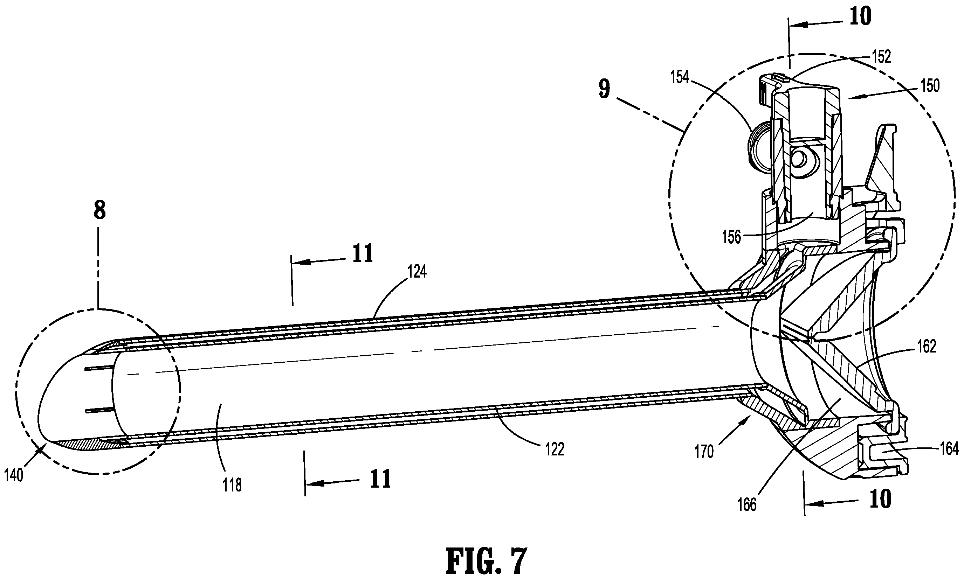

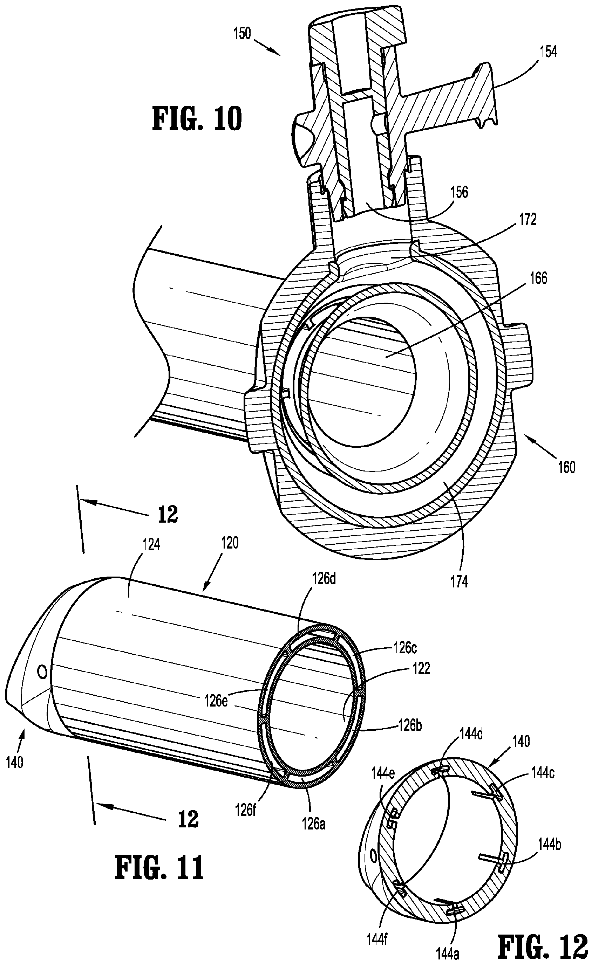

The housing 160 has open proximal and distal ends defining a cavity 166 therein. The proximal opening has a larger diameter than the distal opening. A duck bill or zero-closure seal 162 is positioned in the cavity 166 of the housing 160 (FIG. 7). The zero-closure seal 162 is formed from a suitable resilient material (e.g., silicone) and is configured to prevent fluids from exiting proximally through the housing 160 in the absence of a surgical instrument (e.g., an endoscope) inserted therethrough. The zero-closure seal 162 is sandwiched between the housing 160 and a proximally positioned cap 164. The cap 164 is attached to the housing 160 to retain the zero-closure seal 162 in position and provide a fluid-tight boundary for the housing 160. The cap 164 may be attached to the housing 160 using ultrasonic or RF welding, adhesives, or any other suitable technique for the materials involved. The housing 160 further includes a port 168 having an opening 169 therethrough with a valve 150 positioned therein. The valve 150 has a lever 152 that is rotatable about an axis of the valve 150 allowing the user to open and close the valve 150. The lever 152 is rotatable between an open position of the valve 150 and a closed position of the valve 150. The lever 152 may be positioned in one of a plurality of intermediate positions allowing the user to adjust the flow rate of a fluid through the valve 150. With additional reference to FIGS. 4, 7, and 10, the valve 150 is fluidly coupled to an annular conduit 174 in the collar 170. In particular, the valve 150 is positioned in the opening 169 of port 168 and is aligned with an orifice 172 of the collar 170. This alignment allows fluid to flow through the valve 150, the orifice 172, and into the annular conduit 174. In turn, the annular conduit 174 is open at the proximal end of the collar 170 for fluidly coupling with lumens 126a-f in the tubular member 120 (FIGS. 7 and 10) as will be described in detail hereinbelow.

Referring now to FIGS. 1-4, 7, and 11, the tubular member 120 extends distally from the collar 170 and is formed of a suitable biocompatible material. The tubular member 120 is attached to the collar 170 using known techniques such as RF welding, ultrasonic welding, adhesives, etc. The tubular member 120 may be partially or completely transparent, translucent, or opaque. A passage or channel 118 extends between open proximal and distal ends of the tubular member 120. As illustrated, the tubular member 120 has substantially uniform inner and outer diameters. It is contemplated that either the inner diameter or the outer diameter may vary along a length of the tubular member 120 such that the tubular member 120 is tapered with one of the proximal or distal ends having different diameters from the other of the proximal or distal ends. It is further contemplated that the outer diameter of the tubular member 120 may be tapered such that the distal end has a smaller outer diameter than the proximal end while the inner diameter of the tubular member 120 does not vary along the length of the tubular member 120.

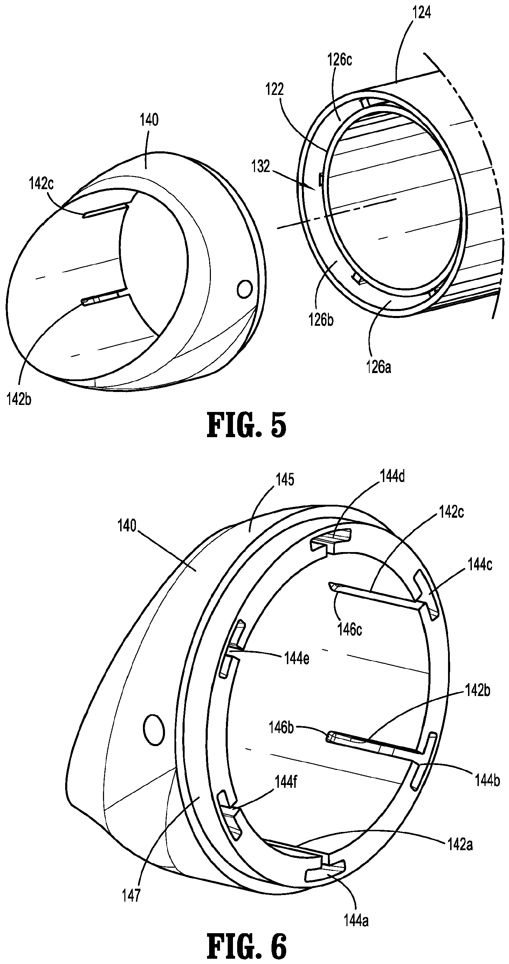

Further, the tubular member 120 has lumens 126a-f defined between an inner wall 122 of the tubular member 120 and an outer wall 124 of the tubular member 120. Each lumen 126 extends longitudinally along a length of the tubular member 120. The inner and outer walls 122, 124 have substantially the same length, but are axially staggered such that a recess 132 is defined in the distal region of the tubular member 120 (FIG. 5) and an extension 134 is defined in the proximal region of the tubular member 120 (FIG. 4). The number of lumens 126 disposed between the inner and outer walls 122, 124 of the tubular member 120 may vary. In embodiments, there may be as few as one or two lumens 126 and in other embodiments, there may be as many as six lumens 126 as illustrated in FIG. 3. However, this does not preclude a greater number of lumens 126 being defined between the inner and outer walls 122, 124 of the tubular member 120.

Each lumen 126 is fluidly coupled to the annular conduit 174 of the collar 170 such that fluid may be supplied to the lumens from a source of fluid FS (FIG. 1) that is coupled to the valve 150 using tubing T. The outlet 156 of the valve 150 is fluidly coupled to the annular conduit 174 via the orifice 172. The fluid may be a cleaning fluid including, but not limited to, an insufflation fluid (e.g., CO₂), sterile saline, a surfactant solution, etc. The fluid flow may be through the valve 150 towards the lumens 126a-f or through the valve 150 towards the source of fluid FS as determined by the differential pressure between the lumens 126a-f and an inlet 154 of the valve 150.

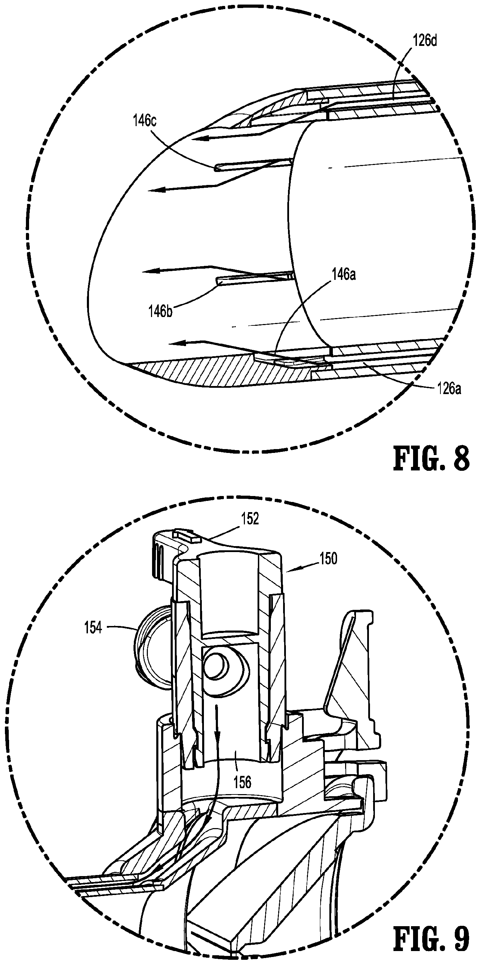

The tip member 140 is located at the distal end of the tubular member 120. With additional reference to FIGS. 5, 6, and 12, the tip member 140 includes a number of ports 142a-f equal to the number of lumens 126a-f of the tubular member 120. Each port 142 includes a duct 144 that is fluidly coupled to a corresponding lumen 126 of the tubular member 120. Each duct 144 extends longitudinally through the tip member 140 and fluidly couples one of the lumens 126 with an outlet 146 of the port 142. Each outlet 146 is configured to direct fluid to a predetermined or target region in the tip member 140 such that the output from each port 142 is directed to the same predetermined region resulting in an increase in the volume of fluid in the predetermined region. One or more of the outlets 146 may be configured to generate turbulent fluid flow. As shown in FIG. 8, a surface of the duct 144 of each port 142 is angled with respect to a longitudinal axis of the tubular member 120 which functions to direct the fluid from the duct 144 to the outlet 146 of the port 142 towards the predetermined region. The tip member 140 has a proximally extending portion 147 with an outer diameter is less than an outer diameter of a body 145 of tip member 140 and the proximally extending portion 147 is receivable in the recess 132 of the tubular member 120 (FIGS. 5 and 6). A distal portion of the tip member 140 is angled such that one location extends further distally than another location (FIG. 5). The tip member 140 is attached to the tubular member 120 using known techniques such as RF welding, ultrasonic welding, adhesives, etc. It is envisioned that one lumen 126 may be fluidly coupled to a plurality of ports 142. In one non-limiting example, the tubular member 120 may include three lumens 126a-c that are fluidly coupled to six ports 142a-f where each lumen 126 is coupled to two ports 142. Other combinations of lumens 126 and ports 142 are also possible.

In the illustrated embodiment with six ports, each port 142 is radially offset by 60° from the adjacent ports 142. In instances where greater or fewer than six ports are disposed in the tip member 140, the amount of radial offset of each port 142 from an adjacent port 142 may be defined by dividing 360° by the number of ports 142 in the distal tip (e.g., four ports would be radially offset by 90° and three ports would be radially offset by 120°). It is contemplated that the radial offset between ports 142 may not be uniform to create a different spray pattern of fluid (e.g., four ports that are radially offset by 30°).

It is contemplated that the ports 142 may not be in the same plane. In particular, one port 142 may be closer to the outer wall 122 while an adjacent port 142 may be closer to the inner wall 124 such that the ports 142 are not on the same plane. It is also contemplated that this staggered arrangement may be repeated for all the ports 142 where one or more ports are on on plane while other ports 142 are on different planes (e.g., three ports located on three different planes). Other combinations of non-planar ports are also envisioned. Further, the ports 142 may be arranged in a helical pattern and the ports 142 may be angled with respect to the longitudinal axis of the surgical access device 100 to provide a desired spray pattern. Additionally, the ports 142 may be staggered longitudinally.

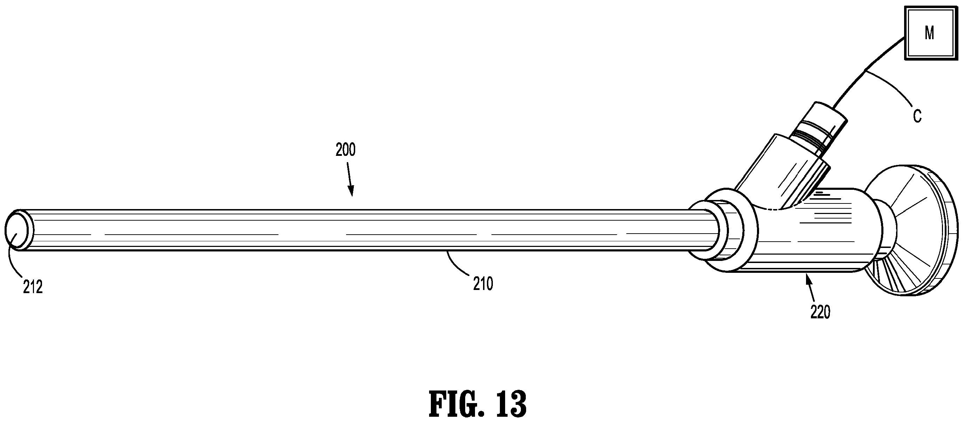

The fluid flow in the predetermined region is usable to remove debris from an outer surface of a lens of a minimally invasive viewing instrument or an endoscope 200 (FIG. 13). The endoscope 200 has a housing 220 with a shaft 210 extending therefrom. A viewing element or lens 212 is located at the distal end of the shaft 210. A monitor M is coupled to the housing 220 of the endoscope 200 using cable C. The monitor M allows the clinician to see what is within the field of view of the lens 212 of the endoscope 200. This allows the clinician to observe the surgical site. During a surgical procedure, the endoscope 200 extends through the surgical access device 100 such that the lens 212 is in position in the surgical site providing the clinician with a view of the surgical site on the monitor M. When the lens 212 of the endoscope 200 is to be cleaned, the clinician moves the lens 212 of the endoscope 200 from the surgical site into the chamber 118 of the tubular member 120 such that an outer surface of the lens 212 is in the predetermined region such that the fluid directed into the predetermined region by the outlets 146a-f of the ports 142a-f impinges upon the outer surface of the lens 212 to gently dislodge particulate debris without damaging the outer surface of the lens 212. Additionally or alternatively, the clinician may move the endoscope 200 distally and proximally into and out of the predetermined region to assist removing debris from the lens 212. During the movement of the endoscope 200, the clinician may check the monitor to locate the position of the lens 212 relative to the predetermined region. This allows the clinician to more accurately position the lens 212 of the endoscope 200 for cleaning and also determine when the lens 212 of the endoscope is sufficiently cleaned. This may be performed with or without a change in the flow rate of fluid into the predetermined region to assist in cleaning debris from the lens 212. This cleans the outer surface of the lens 212 such that the clinician has an unobstructed view through the lens 212 of the endoscope 200. This arrangement allows the clinician to clean the lens 212 of the endoscope 200 without removing the endoscope 200 from the surgical site. As cleaning the lens 212 of the endoscope 200 may occur dozens of times during a surgical procedure, being able to clean the lens 212 without removing the endoscope 200 from the access device will streamline the surgical procedure allowing the clinician to perform the surgical procedure more efficiently and in less time as compared to removing the endoscope 200 multiple times during a procedure to clean it. Additionally, allowing the endoscope 200 to remain in the access device for cleaning reduces the risk of damaging the zero closure seal during repeated removals and insertions of the endoscope 200 for cleaning.

As assembled for use, fluid travels from the source of fluid FS through tubing to the inlet of the valve 150. Repositioning the lever 152 of the valve 150 controls the rate of fluid flow through the valve 150 from zero flow (i.e., valve 150 is fully shut) to full flow (i.e., valve 150 is fully open). With the valve 150 either partially or fully open, the fluid flows through the body of the valve 150 and exits the outlet 156 of the valve 150 where it enters the annular conduit 174 of the collar 170. The annular conduit 174 is fluidly coupled to the lumens 126a-f defined between the inner and outer walls 122, 124 of the tubular member 120 such that fluid exiting the outlet 156 of the valve 150 is directed by the annular conduit 174 to the lumens 126a-f and ultimately to the outlets 146a-f of the ports 142a-f. Although fluid flow is described as traveling from the source of fluid FS to the outlets 146a-f of the ports 142a-f, it is contemplated that fluid may flow from the outlets 146a-f of the ports 142a-f towards the valve 150 and an associated vacuum source or fluid source FS with a lower pressure than the pressure at the outlets 146a-f of the ports 142a-f.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. A surgical access device comprising:
   a housing including a seal;
   a tubular member extending from the housing, the tubular member including a plurality of lumens extending therethrough;
   a valve disposed on the housing and fluidly coupled with a first lumen of the plurality of lumens; and
   a tip member disposed at a distal end of the tubular member, the tip member including a first port that is aligned and fluidly coupled with the first lumen of the plurality of lumens, the first port configured to direct a fluid towards a predetermined location.
2. The surgical access device of paragraph 1, wherein the tubular member includes an inner tube and an outer tube defining an annular chamber therebetween, the annular chamber fluidly coupled to the valve, the first lumen of the plurality of lumens disposed within the annular chamber.
3. The surgical access device of paragraph 3, wherein the annular chamber includes the second lumen of the plurality of lumens extending therethrough, the second lumen of the plurality of lumens fluidly coupled to a second port located in the tip member, the second port configured to direct a fluid towards the predetermined location.
4. The surgical access device of paragraph 1, wherein the inner tubular member defines a third lumen of the plurality of lumens extending therethrough.
5. The surgical access device of paragraph 3, wherein the first and second lumens of the plurality of lumens are radially spaced apart.
6. The surgical access device of paragraph 3, wherein the predetermined location lies along a central longitudinal axis of the tubular member.
7. The surgical access device of paragraph 2, wherein the valve fluidly couples a source of fluid to the first and second lumens of the plurality of lumens.
8. The surgical access device of paragraph 5, wherein the first port is offset from the second port by 180°.
9. The surgical access device of paragraph 8, wherein each of the first and second ports has a spray pattern that covers 180° of the predetermined location.
10. The surgical access device of paragraph 5, wherein the first port and the second port are radially offset in a range between about 60° and about 120°.
11. The surgical access device of paragraph 4, wherein the channel is configured to receive a viewing instrument therethrough.
12. The surgical access device of paragraph 3, wherein the surgical access device is insertable through an opening in tissue.
13. A method for cleaning a viewing instrument comprising:
   moving a lens of a viewing instrument towards a target area defined in a channel of a tubular member, the tubular member including an inner tube disposed in an outer tube defining an annular chamber therebetween; and
   dispensing a cleaning fluid from a first port towards the target area, the first port located on a tip member, the tip member located at a distal end of the tubular member, the first port fluidly coupled to a first lumen of a plurality of lumens that is disposed in the annular chamber, the first lumen of the plurality of lumens fluidly coupled to a valve for controlling flow of the cleaning fluid.
14. The method of paragraph 13, wherein dispensing the cleaning fluid includes dispensing the cleaning fluid from a second port towards the target area, the second port located on the tip member and fluidly coupled to a second lumen of the plurality of lumens that is disposed in the annular chamber, the second lumen of the plurality of lumens fluidly coupled to the valve for controlling flow of the cleaning fluid.
15. The method of paragraph 13, wherein moving the optical portion includes moving the optical portion into a third lumen of the plurality of lumens defined by the inner tube.
16. The method of paragraph 13, further including:
   positioning the tubular member through tissue of a patient, the tubular member extending from a housing with a seal member.
17. The method of paragraph 13, further including repositioning the lens of the viewing instrument along a longitudinal axis of the tubular member such that the lens moves into and out of the predetermined region.
18. The method of paragraph 17, further including viewing an image on a monitor coupled to the viewing instrument during repositioning of the lens.

## Claims

1. A surgical access device comprising:
a housing including a seal;
a tubular member extending from the housing, the tubular member including a plurality of lumens extending therethrough;
a valve disposed on the housing and fluidly coupled with a first lumen of the plurality of lumens; and
a tip member disposed at a distal end of the tubular member, the tip member including a first port that is aligned and fluidly coupled with the first lumen of the plurality of lumens, the first port configured to direct a fluid towards a predetermined location.

2. The surgical access device of claim 1, wherein the tubular member includes an inner tube and an outer tube defining an annular chamber therebetween, the annular chamber fluidly coupled to the valve, the first lumen of the plurality of lumens disposed within the annular chamber.

3. The surgical access device of claim 2, wherein the annular chamber includes the second lumen of the plurality of lumens extending therethrough, the second lumen of the plurality of lumens fluidly coupled to a second port located in the tip member, the second port configured to direct a fluid towards the predetermined location.

4. The surgical access device of any preceding claim, wherein the inner tubular member defines a third lumen of the plurality of lumens extending therethrough.

5. The surgical access device of claim 3 or claim 4, wherein the first and second lumens of the plurality of lumens are radially spaced apart.

6. The surgical access device of claim 3, wherein the predetermined location lies along a central longitudinal axis of the tubular member.

7. The surgical access device of claim 2, wherein the valve fluidly couples a source of fluid to the first and second lumens of the plurality of lumens.

8. The surgical access device of claim 5, wherein the first port is offset from the second port by 180°.

9. The surgical access device of claim 8, wherein each of the first and second ports has a spray pattern that covers 180° of the predetermined location.

10. The surgical access device of claim 5, wherein the first port and the second port are radially offset in a range between about 60° and about 120°.

11. The surgical access device of claim 4, wherein the channel is configured to receive a viewing instrument therethrough.

12. The surgical access device of claim 3, wherein the surgical access device is insertable through an opening in tissue.

13. A method for cleaning a viewing instrument comprising:
moving a lens of a viewing instrument towards a target area defined in a channel of a tubular member, the tubular member including an inner tube disposed in an outer tube defining an annular chamber therebetween; and
dispensing a cleaning fluid from a first port towards the target area, the first port located on a tip member, the tip member located at a distal end of the tubular member, the first port fluidly coupled to a first lumen of a plurality of lumens that is disposed in the annular chamber, the first lumen of the plurality of lumens fluidly coupled to a valve for controlling flow of the cleaning fluid.

14. The method of claim 13, wherein dispensing the cleaning fluid includes dispensing the cleaning fluid from a second port towards the target area, the second port located on the tip member and fluidly coupled to a second lumen of the plurality of lumens that is disposed in the annular chamber, the second lumen of the plurality of lumens fluidly coupled to the valve for controlling flow of the cleaning fluid; and/or wherein moving the optical portion includes moving the optical portion into a third lumen of the plurality of lumens defined by the inner tube; preferably further including:
positioning the tubular member through tissue of a patient, the tubular member extending from a housing with a seal member.

15. The method of claim 13 or claim 14, further including repositioning the lens of the viewing instrument along a longitudinal axis of the tubular member such that the lens moves into and out of the predetermined region; preferably further including viewing an image on a monitor coupled to the viewing instrument during repositioning of the lens.
